# EUROPÄISCHE PATENTSCHRIFT

(11) **EP 3 836 869 B1**
(45) Veröffentlichungstag und Bekanntmachung des Hinweises auf die Patenterteilung: **14.06.2023**
(21) Anmeldenummer: 19758615.9
(22) Anmeldetag: 29.07.2019
(51) Int. Cl.: A61F 5/01, B25J 9/00

(54) **VORRICHTUNG ZUM UNTERSTÜTZEN WENIGSTENS EINES ARMES EINES BENUTZERS**
DEVICE FOR SUPPORTING AT LEAST ONE ARM OF A USER
DISPOSITIF DESTINÉ À SOUTENIR AU MOINS UN BRAS D'UN UTILISATEUR

(30) Priorität: 14.08.2018 DE 102018119754
(43) Veröffentlichungstag der Anmeldung: 23.06.2021
(73) Patentinhaber: Ottobock SE & Co. KGaA, 38122 Braunschweig (DE)
(72) Erfinder: PAPP, Emese, 01099 Dresden (DE)
(74) Vertreter: Gramm, Lins & Partner Patent- und Rechtsanwälte PartGmbB
(86) Internationale Anmeldenummer: PCT/EP2019/070344
(87) Internationale Veröffentlichungsnummer: WO 2020/035298

(56) Entgegenhaltungen:
- DE-A1-102016 121 203
- JP-A- 2009 106 270
- US-A1- 2014 158 839
- US-A1- 2016 339 583

## Beschreibung

Die Erfindung betrifft eine Vorrichtung zum Unterstützen wenigstens eines Armes eines Benutzers, wie in den Ansprüchen definiert.

Eine ähnliche Vorrichtung ist beispielsweise ist aus der US 2016/0081871 A1 sowie der nicht vorveröffentlichten DE 10 2017 112 436 A1 bekannt. Sie verfügt über ein Gegenlagerelement, das in Form eines um den Rumpf des Benutzers herumlegbaren Gurtes ausgebildet ist. An ihm befinden sich zwei entlang des Rückens zur Schulter verlaufende Stützstreben, die oberhalb und seitlich neben den Schultern des Benutzers mit jeweils einem Gelenk verbunden sind, so dass der Arm angehoben werden kann. An den entsprechenden Gelenken sind Federelemente angeordnet, durch die eine nach oben gerichtete Kraft auf die Armschalen ausgeübt werden kann, so dass beispielsweise beim Heben schwerer Gegenstände oder beim Arbeiten über Kopf eine Unterstützung der Arme erfolgen kann. Sollen die Arme gesenkt werden, muss durch die Arme ein Druck auf die Armschalen ausgeübt werden, der die von den Federelementen aufgebrachte Kraft übersteigt, so dass die Arme absinken.

Aus der WO 2014/093408 A2 und der US 9,427,865 B2 ist eine ähnliche Vorrichtung bekannt, bei der als mechanischer Energiespeicher, der als passiver Aktuator wirkt, jeweils eine Feder, insbesondere eine Zugfeder vorgesehen ist. Diese ist mit einem Bowdenzug verbunden. Der Bowdenzug wird über eine Umlenkrolle geführt, so dass beim Verschwenken eines Armes, was eine Bewegung des Armstützelementes relativ zum Gegenlagerelement bedeutet, die Feder gedehnt wird, so dass der mechanische Energiespeicher mit Energie aufgeladen wird. Weitere Unterstützungsvorrichtungen, die insbesondere beim Heben von schweren Gegenständen oder beim Arbeiten über Kopf unterstützen, sind aus der WO 2014/195373 A1, der US 2014/0158839 A1 und der US 2016/339583 A1 bekannt. Diese Vorrichtungen sind jedoch nur auf besondere Bewegungen, die es zu unterstützen gilt, ausgerichtet.

Aus der JP 2009-106270 A ist eine Vorrichtung bekannt, deren Gegenlager ohne Kraftübertragungselement auskommt. Der passive Aktuator ist in Form mehrerer elastischer Stäbe direkt am Gegenlagerelement angeordnet.

Aus der WO 2014/093408 ist eine Unterstützungsvorrichtung bekannt, bei der ein Kraftübertragungselement, das die auftretenden Kräfte in einen Hüftgurt oder Beckengurt ableiten soll, einen elastischen Anteil aufweist, um Bewegungen des Rumpfes folgen zu können. Das Kraftübertragungselement ist an der Vorderseite des Rumpfes des Trägers eingeordnet und wird daher gestaucht oder umgeformt, wenn sich der Träger der Vorrichtung beispielsweise nach vorn beugt.

Der Erfindung liegt daher die Aufgabe zugrunde, diese Nachteile zu beheben oder zu zumindest zu mildern.

Die Erfindung löst die gestellte Aufgabe durch eine Vorrichtung zum Unterstützen zweier Arme eines Benutzers gemäß Anspruch 1, die sich dadurch auszeichnet, dass das Kraftübertragungselement wenigstens ein erstes Bauteil und ein zweites Bauteil aufweist, die durch wenigstens ein arretierbares Gelenk miteinander verbunden sind und dass das Kraftübertragungselement drehbar und/oder schwenkbar an dem Gegenlagerelement angeordnet ist. Durch die Arretierbarkeit des wenigstens einen Gelenkes wird erreicht, dass die beiden Bauteile aneinander festgelegt werden können und sich in diesem Zustand möglichst nicht mehr relativ zueinander bewegen können.

Besonders vorteilhafterweise handelt es sich bei dem ersten Bauteil und/oder dem zweiten Bauteil um Stangen oder Stäbe, die durch ein Scharnier oder Schwenkgelenk miteinander verbunden sind, das eine einzige Schwenkachse aufweist. Durch das Kraftübertragungselement, das Teil des Gegenlagers ist, ist vorteilhafterweise eine Druckkraft übertragbar. Das Armstützelement soll durch eine Kraft, die von unten wirkt, unterstützt werden. Das Kraftübertragungselement soll die entsprechende Gegenkraft in das Gegenlagerelement übertragen, so dass es Druckkräfte übertragen können muss. Ist dies gewährleistet, wird durch die gelenkige Anordnung der beiden Bauteile des Kraftübertragungselementes aneinander erreicht, dass eine optimale Ausgestaltung des Kraftübertragungselementes je nach Bewegung und/oder Position des Benutzers erreicht wird. Die beiden Bauteile des Kraftübertragungselementes können sich relativ zueinander neu ausrichten, indem eines der Bauteile relativ zum anderen der Bauteile um das Schwenkgelenk herum verschwenkt wird. Dadurch können Abstände zwischen dem Gegenlagerelement und dem am Stützelement verändert und an die jeweilige Position und/oder Bewegung des Benutzers angepasst werden.

Besonders bevorzugt sind das erste Bauteil und/oder das zweite Bauteil längenveränderlich ausgebildet. Dabei kann das jeweils längenveränderliche Bauteil als Teleskopstange ausgebildet sein. Auf diese Weise lässt sich die Vorrichtung besonders leicht an unterschiedlich große Benutzer anpassen, indem durch die Teleskopierbarkeit oder die Längenveränderbarkeit wenigstens eines der Bauteile auch die Gesamtlänge des Kraftübertragungselementes angepasst werden kann. Besonders bevorzugt ist das längenveränderliche erste Bauteil und/oder das längenveränderliche zweite Bauteil in ihrer Länge veränderbar in unterschiedlichen Positionen, vorzugsweise stufenlos, jedoch auch arretierbar, so dass die einmal gewünschte Länge eingestellt werden kann und dann nicht mehr veränderlich ist, ohne einen entsprechenden Feststellmechanismus zu lösen.

Vorteilhafterweise handelt es sich bei dem Gelenk um ein Schwenkgelenk mit einer Schwenkachse.

Besonders bevorzugt ist das wenigstens eine arretierbare Gelenk stufenlos arretierbar. Dies bedeutet, dass die beiden Bauteile, die durch das wenigstens eine arretierbare Gelenk miteinander verbunden sind, in verschiedenen Positionen zueinander arretierbar sind, wobei diese Positionen stufenlos angeordnet sind. Es sind zwischen zwei dieser Positionen folglich keine anderen Positionen vorhanden, in denen das Gelenk nicht arretiert werden kann.

Vorzugsweise verfügt die Vorrichtung über zwei Armstützelemente mit je einer Armschale zum Anlegen an jeweils einen Arm.

In einer besonders bevorzugten Ausgestaltung sind das erste Bauteil und/oder das zweite Bauteil derart mit dem Gelenk verbunden, dass eine Bewegung des jeweiligen Bauteils um seine Längsachse relativ zu dem Gelenk möglich ist. Vorteilhafterweise befindet sich am oberen Ende eines der beiden Bauteile, das dem Gegenlagerelement abgewandt ist, das Armstützelement. Das Armstützelement und insbesondere ein Abstandselement des Armstützelementes ist vorteilhafterweise schwenkbar an dem jeweiligen Bauteil des Kraftübertragungselementes angeordnet. Wird dieses Bauteil, an dem sich das Armstützelement befindet, relativ zum Gelenk zwischen dem ersten Bauteil und dem zweiten Bauteil um seine Längsachse drehbar ausgestaltet, kann auch das Armstützelement drehbar um die Längsachse des jeweiligen Bauteils ausgebildet sein. Es werden damit weitere Bewegungen möglich, so dass die Position des Armstützelementes und insbesondere die Erstreckung und gegebenenfalls Form des jeweiligen Kraftübertragungselementes an die Position und/oder Bewegung des Benutzers der Vorrichtung angepasst werden kann.

Erfindungsgemäß ist das Kraftübertragungselement drehbar und/oder schwenkbar an dem Gegenlagerelement angeordnet. Dazu kann das Kraftübertragungselement beispielsweise an dem Gegenlagerelement durch ein Scharnier, ein Kugelgelenk oder ein sonstiges Gelenk befestigt oder beispielsweise mit einem Ende des Kraftübertragungselementes in eine dafür vorgesehene Tasche oder Halterung am Gegenlagerelement eingesteckt oder eingeführt sein. In einer besonders bevorzugten Ausgestaltung ist folglich eines der Bauteile des Kraftübertragungselementes drehbar und/oder schwenkbar am Gegenlagerelement angeordnet und das jeweilige Bauteil um seine eigene Längsachse drehbar relativ zu dem Gelenk angeordnet, durch das das jeweilige Bauteil mit dem jeweils anderen Bauteil des Kraftübertragungselementes verbunden ist. Besonders bevorzugt ist auch dieses andere Bauteil um seine eigene Längsachse drehbar am Gelenk zwischen den beiden Bauteilen befestigt. Dadurch wird eine umfassende Bewegung der Bauteile des Kraftübertragungselementes relativ zueinander, relativ zum Gegenlagerelement und vorzugsweise auch relativ zum Armstützelement möglich. Das Armstützelement verfügt über die Armschiene, in die der Arm eingelegt wird. Diese Armschiene lässt sich durch die vielseitig positionierbare und drehbare sowie verschwenkbare Ausgestaltung des Kraftübertragungselementes in eine Vielzahl unterschiedlicher Positionen bringen, wodurch nicht nur eine Bewegung des eigentlichen Schultergelenkes, also eines Kugelgelenkes, gefolgt werden kann, sondern auch andere Bewegungen möglich werden, die eine Bewegung der gesamten Schulter zur Folge haben.

Vorzugsweise ist das Gegenlagerelement ein Anlageelement zum Anlegen an den Rumpf des Benutzers. Es kann ein Gurt, ein Gürtel, eine Bandage oder ein Schalenelement sein und kann vorteilhafterweise in ein Kleidungsstück, beispielsweise eine Hose oder eine Weste integriert sein. Alternativ oder zusätzlich dazu weist das Gegenlagerelement ein Schulterelement zum Anlegen an einer Schulter des Benutzers auf.

Vorzugsweise ist der Kraftangriffshebel drehfest mit einem der Armstützelemente oder dem Kraftübertragungselement verbunden ist schließt mit dem jeweiligen Element einen Winkel, der einstellbar ist.

Durch den wenigstens einen passiven Aktuator kann eine Kraft auf das Armstützelement ausgeübt werden, wobei diese Kraft an einem Kraftangriffshebel angreift. Der Kraftangriffshebel ist in einer ersten Ausgestaltung mit einem der Armstützelemente, auf die vom Aktuator aufbringbare Kraft ausgeübt werden soll, drehfest verbunden. In diesem Fall kann beispielsweise der Aktuator eine Feder oder ein elastisches Element sein, dessen eines Ende beispielsweise am Gegenlager angeordnet ist. Das andere Ende greift am Kraftangriffshebel an. Vorteilhafterweise ist das Armstützelement schwenkbar am Kraftübertragungselement angeordnet. Da in dieser ersten Ausgestaltung der Kraftübertragungshebel drehfest am Armelement angeordnet ist, ist folglich auch der Kraftübertragungshebel schwenkbar am Kraftübertragungselement befestigt. Durch die vom passiven Aktuator auf den Kraftübertragungshebel wirkende Kraft wird folglich ein Drehmoment auf das Armstützelement und damit eine unterstützende Kraft ausgeübt. Dieses Drehmoment ist am größten, wenn der Kraftübertragungshebel senkrecht auf der Richtung der wirkenden Kraft steht.

Hebt ein Benutzer einer derartigen Vorrichtungen den Arm, wird das Armstützelement und damit auch der drehfest mit ihm verbundene Kraftangriffshebel um das Kraftübertragungselement herum verschwenkt. Der Winkel zwischen dem Kraftangriffshebel und dem Kraftübertragungselement verändert sich folglich. Gleichzeitig verändert sich auch der Winkel zwischen dem Kraftangriffshebel und der Wirkrichtung der durch den wenigstens einen passiven Aktuator aufbringbaren Kraft. Bei einer bestimmten Stellung der Arme steht der Kraftangriffshebel senkrecht auf der Richtung der wirkenden Kraft. In diesem Moment ist das durch den Aktuator auf den Kraftangriffshebel und damit auf das Armstützelement aufgebrachte Drehmoment am größten und somit die Unterstützungskraft für den Arm und das Armstützelement maximal.

In dieser Ausgestaltung ist nun vorgesehen, dass der Winkel zwischen dem Armstützelement und dem Kraftangriffshebel einstellbar ist. Der Winkel kann folglich verändert werden und ist in wenigstens zwei, vorzugsweise vielen, insbesondere vorteilhaft stufenlos, feststellbar, so dass eine drehfeste Verbindung zwischen dem Kraftangriffshebel und dem Armstützelement erreicht wird. Durch die Einstellung dieses Winkels lässt sich die Position der Arme des Benutzers verändern, in der der Kraftangriffshebel senkrecht auf der durch den wenigstens einen Aktuator aufbringbaren Kraft steht.

In einer anderen Ausführungsform ist der Kraftangriffshebel drehfest am Kraftübertragungselement befestigt. Auch in diesem Fall kann der Aktuator beispielsweise eine Feder oder ein elastisches Element sein, dessen eines Ende nun jedoch nicht am Kraftübertragungselement oder dem Gegenlager angeordnet ist, sondern mit dem Armstützelement verbunden ist. Der Aktuator ist mit dem anderen Ende mit dem Kraftangriffshebel verbunden. Auch in diesem Fall kann durch den wenigstens einen passiven Aktuator ein Drehmoment auf den Kraftangriffshebel ausgeübt werden, so dass eine unterstützende Kraft auf das Armstützelement ausgeübt wird. Auch in diesem Fall ist es von Vorteil, wenn das Armstützelement drehbar am Kraftübertragungselement angeordnet ist.

Auch in dieser Ausgestaltung ist das durch den wenigstens einen passiven Aktuator erzeugte Drehmoment und damit die auf das Armstützelement ausübbare Kraft maximal, wenn der Winkel zwischen dem Kraftangriffshebel und der durch den Aktuator ausübbaren wirkenden Kraft 90° ist. Ist nun der Winkel zwischen dem Kraftangriffshebel und dem Kraftübertragungselement einstellbar, lässt sich auch in dieser Ausgestaltung diese Position, in der das Drehmoment und damit die Unterstützungskraft maximal ist, verändern.

Wird bezüglich der vorliegenden Anmeldung von einer Richtung des Kraftangriffshebels oder einem Winkel zwischen einem Bauteil und dem Kraftangriffshebel gesprochen, ist mit dieser Richtung immer die Richtung vom Kraftangriffspunkt, also dem Punkt, an dem die vom Aktuator ausübbare Kraft am Kraftangriffshebel angreift, und dem Drehpunkt gemeint, um den das Armstützelement relativ zum Kraftübertragungselement schwenkbar ist.

In einer bevorzugten Ausgestaltung der vorliegenden Erfindung ist der Kraftangriffshebel in einem ersten Zustand, in dem er relativ zu dem Armstützelement und dem Kraftübertragungselement schwenkbar ist, und in einen zweiten Zustand bringbar, in dem er drehfest mit dem Armstützelement oder dem Kraftübertragungselement angeordnet ist. Während sich der Kraftangriffshebel im ersten Zustand befindet, wird ein Winkel zwischen dem Kraftangriffshebel und dem Armstützelement oder dem Kraftangriffshebel und dem Kraftübertragungselement einstellbar. Er kann durch Verschwenken des Kraftangriffshebels verändert werden. Ist der gewünschte Winkel eingestellt, wird der Kraftangriffshebel in den zweiten Zustand überführt, so dass er nicht mehr relativ zum Armstützelement und dem Kraftübertragungselement verdreht werden kann.

In einer bevorzugten Ausgestaltung verfügt das Kraftübertragungselement und/oder das Armstützelement über zwei Bauteile, die durch ein Schwenkgelenk miteinander verbunden sind, so dass ein Winkel zwischen diesen beiden Bauteilen einstellbar ist. Vorteilhafterweise ist das Schwenkgelenk lösbar und feststellbar. Durch diese Ausgestaltung des Schwenkgelenkes kann eine Richtung des Kraftübertragungselementes verändert werden. Die Richtung des Kraftübertragungselementes, die beispielsweise wichtig ist, um den Winkel zwischen dem Kraftübertragungselement und dem Kraftangriffshebel zu bestimmen, ist die Richtung zwischen dem Drehpunkt, um den das Armstützelement relativ zum Kraftübertragungselement verschwenkbar ist, und dem Lagerpunkt, an dem das Kraftübertragungselement am Gegenlagerelement angeordnet ist.

In einer bevorzugten Ausgestaltung handelt es sich bei dem Gegenlagerelement des Gegenlagers beispielsweise um einen Hüftgurt, der vom Benutzer der Vorrichtung um die Hüfte oder den Bauch herum getragen wird. Das Kraftübertragungselement ist beispielsweise eine aus einem oder mehreren Bauteilen bestehende Stange, wobei zwischen mehreren Bauteilen der Stange ein oder mehrere Schwenkgelenke angeordnet sein können, durch die der Winkel zwischen den einzelnen Bauteilen einstellbar aber feststellbar ist. Dieses Kraftübertragungselement ist mit einem Ende am Gegenlagerelement befestigt. Am anderen Ende ist um ein Schwenkgelenk schwenkbar das Armstützelement angeordnet, das beispielsweise ein Abstandselement in Form einer Stange, die beispielsweise teleskopierbar oder nicht teleskopierbar ausgebildet sein kann. Alle Richtungen, die zur Bestimmung des Winkels, der erfindungsgemäß einstellbar sein soll, verwendet werden, sind in Richtung auf den jeweiligen Drehpunkt dieses Schwenkgelenkes gerichtet. Ist der Kraftangriffshebel drehfest mit dem Armstützelement verbunden, wird der Winkel gebildet zwischen der Richtung zwischen dem Kraftangriffspunkt am Kraftangriffshebel und dem Drehpunkt des Schwenkgelenkes einerseits und der Richtung des Kraftübertragungselementes andererseits, die durch die Verbindungslinie zwischen dem Drehpunkt des Schwenkgelenkes einerseits und dem Lagerpunkt andererseits gebildet wird, an dem das Kraftübertragungselement am Gegenlager positioniert ist.

Ist hingegen der Kraftangriffshebel drehfest mit dem Kraftübertragungselement verbunden, wird der Winkel bestimmt zwischen der Richtung des Kraftangriffshebels, also der Richtung zwischen dem Kraftangriffspunkt und dem Drehpunkt des Schwenkgelenkes einerseits und der Richtung des Armstützelementes, also der Richtung zwischen der Armschale, in die der Arm eingelegt wird, und dem Drehpunkt des Schwenkgelenkes zwischen Armstützelement und Kraftübertragungselement.

In einer bevorzugten Ausführungsform sind an dem wenigstens einen Gelenk, bevorzugt an allen Gelenken wenigstens ein, bevorzugt zwei Anschläge angeordnet, die einen Bewegungsbereich der beiden Bauteile, die durch das jeweilige Gelenk miteinander verbunden sind, in wenigstens einer Richtung, bevorzugt in allen Richtungen einzuschränken. Sind die Anschläge weit genug verstellbar ausgebildet, lässt sich das jeweilige Gelenk auch durch ein Verstellen der Anschläge arretieren.

Durch die Anschläge kann verhindert werden, dass zwei Bauteile, die durch eines der jeweiligen Gelenke miteinander verbunden sind, soweit relativ zueinander durch das Gelenk bewegt werden, dass die benötigte Stabilität nicht mehr vorhanden ist. Die Anschläge dienen insbesondere also der Betriebssicherheit und Sicherstellung der Funktionalität der Vorrichtung.

In einer bevorzugten Ausgestaltung sind die Anschläge mit Markierungen, Skalierungen oder anderen Elementen ausgebildet, die eine Reproduzierbarkeit der gefundenen Einstellungen erlauben. Ist die optimale Positionierung der Gelenke und damit der unterschiedlichen Bauteile zueinander gefunden, kann beispielsweise bei einem Defekt oder einer Reparatur oder auch zur Reinigung des Systems oder zum Transport das System zusammengeklappt, demontiert oder in eine andere Position gebracht werden, ohne dass die Informationen über die gefundene Positionierung verloren gehen. Auf diese Weise können die Einstellungen beispielsweise auf eine andere baugleiche Vorrichtung übertragen werden oder nach dem Transport, der Reparatur, der erneuten Montage oder sonstigen Vorgängen wieder auf die ursprüngliche Vorrichtung übertragen werden.

Mit Hilfe der beiliegenden Zeichnungen wird nachfolgend ein Ausführungsbeispiel der vorliegenden Erfindung näher erläutert. Es zeigen:
- Figuren 1- 3 -: eine Vorrichtung gemäß einem ersten Ausführungsbeispiel der vorliegenden Erfindung im angelegten Zustand,
- Figur 4 -: eine Vorrichtung gemäß einem zweiten Ausführungsbeispiel der vorliegenden Erfindung,
- Figur 5 -: ein mögliches Kraftübertragungselement,
- Figuren 6a und 6b -: ein weiteres mögliches Kraftübertragungselement in zwei Positionierungen,
- Figuren 7a und 7b -: ein weiteres Ausführungsbeispiel eines Kraftübertragungselementes in zwei Positionen und
- Figur 8 -: die schematische Darstellung einer Vorrichtung gemäß einem weiteren Ausführungsbeispiel der vorliegenden Erfindung.

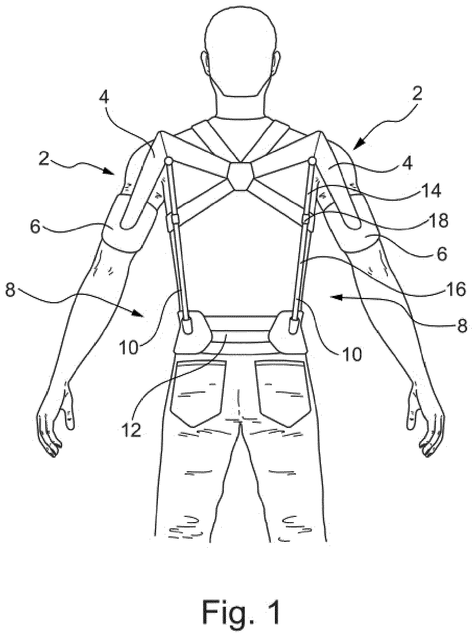

Figur 1 zeigt eine Vorrichtung zum Unterstützen zweier Arme eines Benutzers, wobei die Vorrichtung zwei Armstützelemente 2 aufweist, die jeweils ein Abstandselement 4 und eine Armschale 6 aufweisen. Zudem verfügt die Vorrichtung über zwei Gegenlager 8, die jeweils ein Kraftübertragungselement 10 aufweisen, die mit einem gemeinsamen Gegenlagerelement 12 verbunden sind. Jeder der Kraftübertragungselemente 10 verfügt über ein erstes Bauteil 14 sowie ein zweites Bauteil 16, die durch ein dazwischenliegendes Gelenk 18 miteinander verbunden sind.

In Figur 1 ist der Benutzer der Vorrichtung mit gesenkten Armen dargestellt. Ein Winkel zwischen dem ersten Bauteil 14 und dem zweiten Bauteil 16 beträgt annähernd 180°.

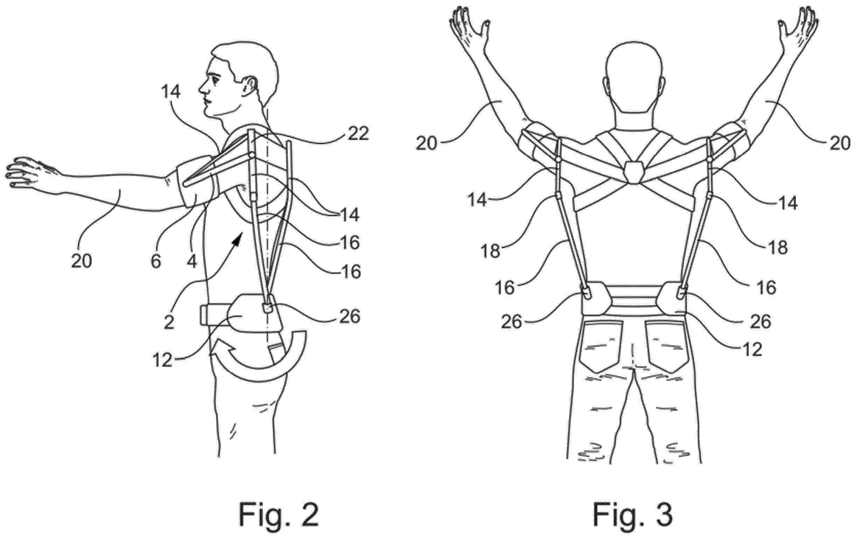

Figur 2 zeigt die Vorrichtung in einer Seitenansicht. Man erkennt den Arm 20 des Benutzers, der nun nach vorne bewegt wurde. Die Armschale 6 ist am Abstandselement 4 angeordnet, das gegenüber dem Kraftübertragungselement 10 schwenkbar ausgebildet ist. Zwischen dem Abstandselement 4 und einem Kraftangriffshebel 22 erstreckt sich ein passiver Aktuator 24, durch den eine Kraft auf das Armstützelement 2 aufgebracht werden kann. Die Position des ersten Bauteils 14 sowie des zweiten Bauteils 16 des Kraftübertragungselementes 10, in der sich die beiden Elemente in Figur 1 befinden, ist rechts in Figur 2 dargestellt. Durch die Bewegung des Arms 20 nach vorne, verschiebt sich die Position und das erste Bauteil 14 sowie das zweite Bauteil 16 werden nach vorne verschoben. Sie bewegen sich nahezu seitlich neben den Benutzer. Dabei wird ein unteres Ende 26 des zweiten Bauteils 16 relativ zum Gegenlagerelement 12 bewegt. Dazu befindet es sich in einer Tasche, die am Gegenlagerelement 12 angeordnet ist. Man erkennt zudem, dass sich ein Winkel zwischen dem ersten Bauteil 14 und dem zweiten Bauteil 16 in der neuen Position, die links in Figur 2 dargestellt ist, verändert hat.

Figur 3 zeigt die Situation, in der der Benutzer beide Arme 20 nach oben hebt. Die ersten Bauteile 14 und die zweiten Bauteile 16 der beiden Kraftübertragungselemente 10 haben erneut neue Positionen relativ zueinander eingenommen und werden über die Gelenke 18 relativ zueinander verschwenkt. Auch die Position der unteren Enden 26 relativ zum Gegenlagerelement 12 hat sich verändert. Dadurch kann allen Bewegungen optimal gefolgt werden, ohne dass es zu Beeinträchtigungen oder störenden Wechselwirkungen der Vorrichtung mit den Armen oder der Schulter kommt.

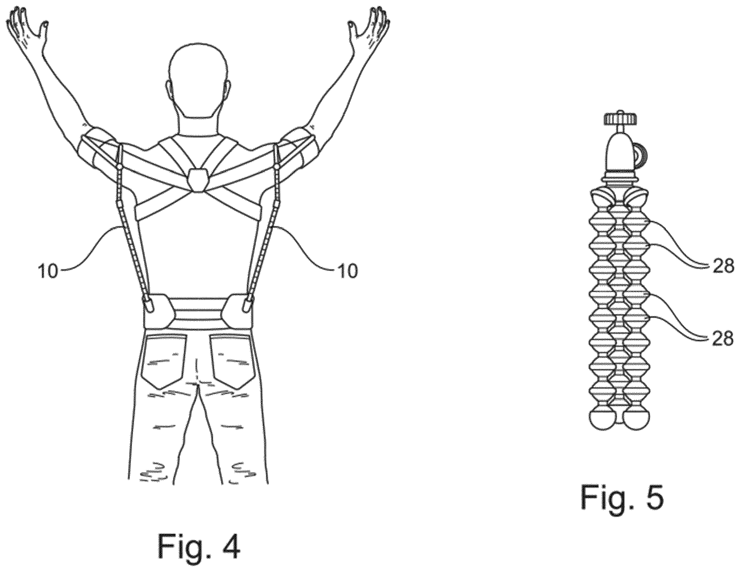

Figur 4 zeigt die Darstellung aus Figur 3 mit einem weiteren Ausführungsbeispiel der vorliegenden Erfindung. Die Kraftübertragungselemente 10 bestehen im gezeigten Ausführungsbeispiel aus einer Vielzahl von Elementen, die jeweils relativ zueinander verschiebbar sind. Es handelt sich um eine versteifbare Gelenkkette, durch die weiterhin Druckkräfte übertragen werden können. Während in der Figur 3 die verwendeten ersten Bauteile 14 und/oder die zweiten Bauteile 16 auch in der Länge veränderbar sein können, indem sie beispielsweise als Teleskopstangen ausgebildet werden, ist eine versteifbare Gelenkkette, wie sie in Figur 5 dargestellt ist, in ihrer Länge begrenzt. Die einzelnen Bauelemente 28 sind durch Gelenke miteinander verbunden und können sich nahezu jeder Form anpassen, ohne dass ihre Fähigkeit, Druckkräfte zu übertragen, darunter leidet.

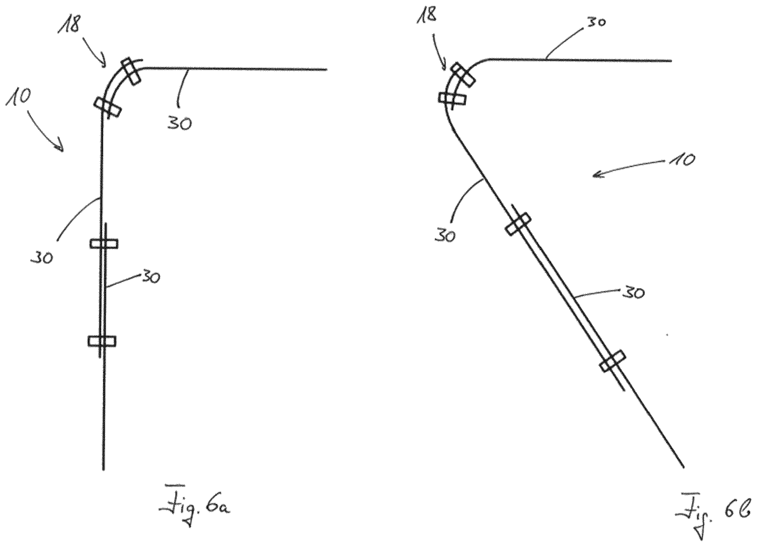

Die Figuren 6a und 6b zeigen jeweils ein Kraftübertragungselement 10, das jeweils aus drei Teilelementen 30 aufgebaut ist. Diese drei Teilelemente sind über jeweils zwei Verbindungselemente paarweise miteinander gekoppelt und verfügen in diesem Bereich jeweils über den gleichen Krümmungsradius. Sie können somit gegeneinander verschoben werden, wodurch das Gelenk 18 realisiert wird. Dadurch lassen sich die in den Figuren 6a und 6b gezeigten unterschiedlichen Krümmungsradien zwischen den beiden Richtungen erstrecken, in die sich das Kraftübertragungselement 10 an seinen beiden Enden erstreckt.

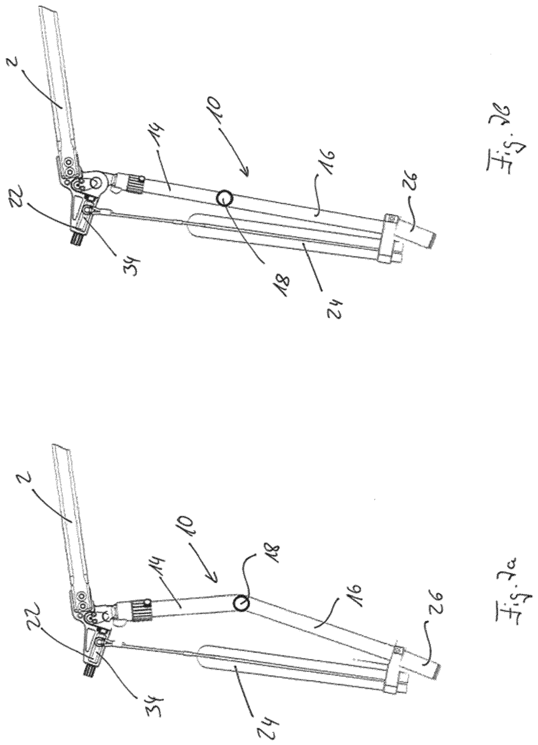

Die Figuren 7a und 7b zeigen ein weiteres Ausführungsbeispiel. Das Kraftübertragungselement 10 verfügt über ein erstes Bauteil 14 und ein zweites Bauteil 16, die durch jeweils 1 Gelenk 18 miteinander verbunden sind. Das Gelenk 18 ist arretierbar ausgebildet.

Am Kraftangriffshebel 22 greift der Aktuator 24 an, der die benötigte Kraft zum Unterstützen des Armstützelementes 2 aufbringt. Man erkennt deutlich, dass der Abstand zwischen den Kraftübertragungshebel 22 und dem unteren Ende 26 des Kraftübertragungselementes 10 in Figur 7a kleiner ist als in Figur 7b. Dies wird durch den Knick, der durch die Einstellung des Gelenks 18 zwischen dem ersten Bauteil 14 und dem zweiten Bauteil 16 in Figur 7a vorhanden ist, hervorgerufen. Es hat sich daher als allgemein vorteilhaft herausgestellt, wenn der Aktuator 24 eine Spannvorrichtung aufweist, die nicht dargestellt ist, jedoch dafür sorgt, dass die Spannung des Aktuators 24 und damit die unterstützende Kraft einstellbar ist, um derartige in Figuren 7a und 7b dargestellte Längenänderungen ausgleichen zu können.

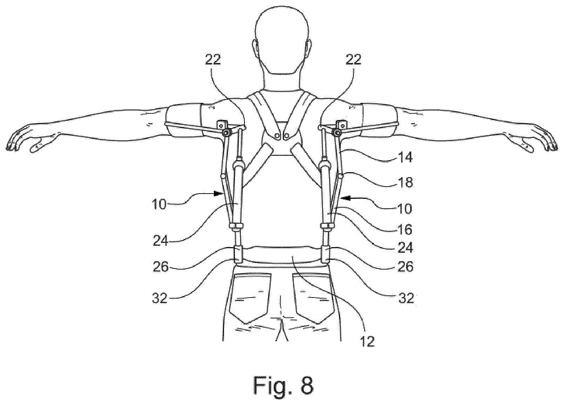

Figur 8 zeigt eine solche Situation. Die beiden Kraftübertragungselemente 10 weisen jeweils ein erstes Bauteil 14 und ein zweites Bauteil 16 auf, die durch jeweils ein Gelenk 18 miteinander verbunden sind. Der besseren Übersichtlichkeit wegen ist dies nur bei dem rechten Element dargestellt. Die Aktuatoren 24 greifen am jeweiligen Kraftangriffshebel 22 an, wobei auch hier der Abstand zwischen dem Kraftübertragungshebel 22 und dem unteren Ende 26 des Kraftübertragungselementes von dem Winkel abhängt, in dem das erste Bauteil 14 und das zweite Bauteil 16 relativ zueinander durch das Gelenk 18 arretiert sind. Man erkennt in Figur 8, dass die unteren Enden 26 in dafür vorgesehenen Aufnahmetaschen 32 angeordnet sind, die am Gegenlagerelement 12, das als Hüftgurt ausgebildet ist, positioniert sind. Die Stelle, an der der jeweilige Aktuator 24 am Kraftübertragungshebel 22 angeordnet ist, ist vorzugsweise einstellbar ausgebildet, indem das in den Figuren gezeigte obere Ende des Aktuators in dem in den Figuren 7a und 7b gezeigten Führungsschlitz 34 verschiebbar angeordnet ist. Selbstverständlich sind auch andere Möglichkeiten der Verschiebbarkeit oder Verstellbarkeit möglich.

### Bezugszeichenliste

- 2 -: Armstützelement
- 4 -: Abstandselement
- 6 -: Armschale
- 8 -: Gegenlager
- 10 -: Kraftübertragungselement
- 12 -: Gegenlagerelement
- 14 -: erstes Bauteil
- 16 -: zweites Bauteil
- 18 -: Gelenk
- 20 -: Arm
- 22 -: Kraftangriffshebel
- 24 -: Aktuator
- 26 -: unteres Ende
- 28 -: Bauelement
- 30 -: Teilelement
- 32 -: Aufnahmetasche
- 34 -: Führungsschlitz

## Patentansprüche

1. Vorrichtung zum Unterstützen wenigstens eines Armes (20) eines Benutzers, wobei die Vorrichtung
a. wenigstens ein Armstützelement (2) mit je einer Armschale (6) zum Anlegen an jeweils einen Arm (20),
b. wenigstens einen passiven Aktuator (24),
i. der eingerichtet ist, eine Kraft auf wenigstens eines der Armstützelemente (2) auszuüben, und
c. wenigstens ein Gegenlager (8) für die aufzubringende Kraft aufweist,
i. das wenigstens ein Kraftübertragungselement (10) und ein Gegenlagerelement (12) aufweist,
wobei das Kraftübertragungselement (10) drehbar und/oder schwenkbar an dem Gegenlagerelement (12) angeordnet ist, und das Kraftübertragungselement (10) wenigstens ein erstes Bauteil (14) und ein zweites Bauteil (16) aufweist, die durch wenigstens ein Gelenk (18) miteinander verbunden sind,
**dadurch gekennzeichnet, dass**
das Gelenk (18) arretierbar ist.

2. Vorrichtung nach Anspruch 1, **dadurch gekennzeichnet, dass** das erste Bauteil (14) und/oder das zweite Bauteil (16) längenveränderlich ausgebildet sind, insbesondere Teleskopstangen sind.

3. Vorrichtung nach Anspruch 1 oder 2, **dadurch gekennzeichnet, dass** das Gelenk (18) ein Schwenkgelenk mit einer Schwenkachse ist.

4. Vorrichtung nach einem der vorstehenden Ansprüche, **dadurch gekennzeichnet, dass** das wenigstens eine arretierbare Gelenk (18) stufenlos arretierbar ist.

5. Vorrichtung nach einem der vorstehenden Ansprüche, **dadurch gekennzeichnet, dass** die Vorrichtung zwei Armstützelemente (2) mit je einer Armschale (6) zum Anlegen an jeweils einen Arm (20) aufweist.

6. Vorrichtung nach einem der vorstehenden Ansprüche, **dadurch gekennzeichnet, dass** das erste Bauteil (14) und/oder das zweite Bauteil (16) derart mit dem Gelenk (18) verbunden sind, dass eine Bewegung des jeweiligen Bauteils (14,16) um seine Längsachse relativ zu dem Gelenk (18) möglich ist.

7. Vorrichtung nach einem der vorstehenden Ansprüche, **dadurch gekennzeichnet, dass** das Armstützelement (2) oder das Kraftübertragungselement (10) einen Kraftangriffshebel (22) aufweist, an dem der Aktuator (24) angreift.

8. Vorrichtung nach Anspruch 7, **dadurch gekennzeichnet, dass** der Kraftangriffshebel (22) drehfest mit einem der Armstützelemente (2) oder dem Kraftübertragungselement (10) verbunden ist und einen Winkel einschließt, der einstellbar ist.

9. Vorrichtung nach Anspruch 7 oder 8, **dadurch gekennzeichnet, dass** ein Kraftangriffspunkt, an dem der Aktuator (24) an dem Kraftangriffshebel (22) angreift, verschiebbar ist.

## Claims

1. A device for supporting at least one arm (20) of a user, wherein the device has
a. at least one arm support element (2), each of which has an arm shell (6) for placing on an arm (20),
b. at least one passive actuator (24),
i. which is configured to apply a force to at least one of the arm support elements (2), and
c. at least one counter bearing (8) for the force to be applied,
i. which comprises at least one force transmission element (10) and a counter bearing element (12),
wherein the force transmission element (10) is arranged rotatably and/or pivotably on the counter bearing element (12), and the force transmission element (10) comprises at least a first component (14) and a second component (16) which are connected to one another by at least one joint (18),
**characterized by the fact that**
the joint (18) is lockable.

2. The device according to claim 1, **characterized by** the fact that the first component (14) and/or the second component (16) are variable in length, in particular are telescopic rods.

3. The device according to claim 1 or 2, **characterized by** the fact that the joint (18) is a swivel joint with a swivel axis.

4. The device according to one of the above claims, **characterized by** the fact that the joint (18) is infinitely lockable.

5. The device according to one of the above claims, **characterized by** the fact that the device comprises two arm support elements (2), each of which has an arm shell (6) for placing on an arm (20).

6. The device according to one of the above claims, **characterized by** the fact that the first component (14) and/or the second component (16) are connected by the joint (18) such that the respective component (14, 16) can be moved around its longitudinal axis relative to the joint (18).

7. The device according to one of the above claims, **characterized by** the fact that the arm support element (2) or the force transmission element (10) comprises a force application lever (22) with which the actuator (24) engages.

8. The device according to claim 7, **characterized by** the fact that the force application lever (22) is connected to one of the arm support elements (2) or to the force transmission element (10) such that it is torque-proof an confines an angle wherein the angle is adjustable.

9. The device according to claim 7 or 8, **characterized by** the fact that a force application point at which the force application lever engages, is movable.

## Revendications

1. Dispositif destiné à soutenir au moins un bras (20) d'un utilisateur, le dispositif comprenant
a. au moins un élément de soutien de bras (2) ayant chacun une coque pour bras (6) destinée à être appliquée contre un bras (20) respectif,
b. au moins un actionneur passif (24),
i. qui est conçu pour exercer une force sur l'un au moins des éléments de soutien de bras (2), et
c. au moins un support antagoniste (8) pour la force à appliquer,
i. qui comprend au moins un élément de transmission de force (10) et au moins un élément de support antagoniste (12),
l'élément de transmission de force (10) étant disposé de façon mobile en rotation et/ou en pivotement sur l'élément de support antagoniste (12), et l'élément de transmission de force (10) comprenant au moins un premier composant (14) et un deuxième composant (16) qui sont reliés entre eux par au moins une articulation (18),
**caractérisé en ce que**
l'articulation (18) est verrouillable.

2. Dispositif selon la revendication 1,
**caractérisé en ce que** le premier composant (14) et/ou le deuxième composant (16) sont de longueur variable, en particulier sont des tiges télescopiques.

3. Dispositif selon la revendication 1 ou 2,
**caractérisé en ce que** l'articulation (18) est une articulation pivotante avec un axe de pivotement.

4. Dispositif selon l'une des revendications précédentes,
**caractérisé en ce que** ladite au moins une articulation verrouillable (18) est verrouillable en continu.

5. Dispositif selon l'une des revendications précédentes,
**caractérisé en ce que** le dispositif comporte deux éléments de soutien de bras (2) comprenant chacun une coque pour bras (6) destinée à être appliquée contre un bras respectif (20).

6. Dispositif selon l'une des revendications précédentes,
**caractérisé en ce que** le premier composant (14) et/ou le deuxième composant (16) sont reliés à l'articulation (18) de manière à permettre un mouvement du composant respectif (14, 16) autour de son axe longitudinal par rapport à l'articulation (18).

7. Dispositif selon l'une des revendications précédentes,
**caractérisé en ce que** l'élément de soutien de bras (2) ou l'élément de transmission de force (10) comporte un levier d'application de force (22) sur lequel l'actionneur (24) agit.

8. Dispositif selon la revendication 7,
**caractérisé en ce que** le levier d'application de force (22) est relié solidairement en rotation à l'un des éléments de soutien de bras (2) ou à l'élément de transmission de force (10) et définit un angle qui est réglable.

9. Dispositif selon la revendication 7 ou 8,
**caractérisé en ce qu'**un point d'application de force, au niveau duquel l'actionneur (24) agit sur le levier d'application de force (22), est déplaçable.
